# EUROPEAN PATENT APPLICATION

(11) **EP 4 299 088 A1**
(43) Date of publication of application: **03.01.2024**
(21) Application number: 23182106.7
(22) Date of filing: 28.06.2023
(51) Int. Cl.: A61M 5/142, A61M 5/145, A61M 5/168, A61M 5/172, A61M 5/20, A61M 5/315

(54) **METHODS AND SYSTEMS FOR OPTICAL-BASED FLUID CONTROL IN A FLUID DELIVERY SYSTEM**

(30) Priority: 28.06.2022 US 202263356056 P
(71) Applicant: Insulet Corporation, Acton MA 01720 (US)
(72) Inventor: ALLIS, Daniel, Boxford, MA (US)
(74) Representative: Peterreins Schley

(57) **Abstract**

Disclosed are systems and processes for an optical monitoring system for monitoring the dispensing of fluid from a fluid delivery device For example, a fluid delivery device may include a reservoir storing a fluid, a pump fluidically coupled to the reservoir, the pump comprising a drive mechanism to force the fluid from the reservoir, and an optical monitoring system. The optical monitoring system may include at least one light source operative to emit light incident on at least one element of the drive mechanism, and at least one sensor configured to receive the light reflecting off of the at least one element, wherein the at least one light source and the at least one sensor are arranged on a same side of the at least one element. Other embodiments are described.

## Description

### FIELD OF THE INVENTION

The present disclosure generally relates to a drug delivery system, and, in particular, improved methods and systems for controlling and measuring fluid delivery in a drug delivery system.

### BACKGROUND

Healthcare providers may prescribe patients wearable fluid delivery devices for delivering fluids, such as liquid medicaments, as part of a treatment regimen. Non-limiting examples of medicaments may include chemotherapy drugs, hormones (for instance, insulin), pain relief medications, and other types of liquid-based drugs. Medicament delivery devices require fine dosage control on a micro-scale to ensure proper and safe dispensing of the medicament according to a dosage prescription and/or patient needs. Conventional systems use regulation systems, such as valves, that have accuracy and repeatability challenges with typical dosage levels. For example, standard available liquid pump systems for wearable fluid delivery devices are not able to accurately measure how much of a medicament was initially loaded into the device leading to confusion on exactly when the reservoir will be completely empty. In another example, standard available liquid pump systems are not able to provide precise indications of how much of the medicament has been delivered to the patient.

It is with considerations of these and other challenges in mind that improvements such as disclosed in the present disclosure may be useful.

### BRIEF DESCRIPTION OF THE DRAWINGS

**FIG. 1** illustrates a first exemplary embodiment of an operating environment in accordance with the present disclosure;
**FIG. 2** illustrates a drive mechanism for a fluid delivery device in accordance with the present disclosure;
**FIG. 3** illustrates an embodiment of a wearable fluid delivery device in accordance with the present disclosure
**FIG. 4** illustrates an example optical monitoring system in accordance with the present disclosure;
**FIG. 5** illustrates an example differential or gradient light reflectance element in accordance with the present disclosure; and
**FIG. 6** illustrates a functional block diagram of a system in accordance with the present disclosure.

The drawings are not necessarily to scale. The drawings are merely representations, not intended to portray specific parameters of the disclosure. The drawings are intended to depict example embodiments of the disclosure, and therefore should not be considered as limiting in scope. In the drawings, like numbering represents like elements.

### DETAILED DESCRIPTION

Various features of drug delivery devices and processes will now be described more fully hereinafter with reference to the accompanying drawings, in which one or more features of the drug delivery devices and processes will be shown and described. It should be appreciated that the various features or the like described hereinafter may be used independently of, or in combination with, each other. It will be appreciated that a drug delivery device and/or process for operating a drug delivery device disclosed herein may be embodied in many different forms and should not be construed as being limited to the embodiments set forth herein. Rather, these embodiments are provided so that this disclosure will convey certain or features of the drug delivery device and/or process for operating the drug delivery device to those skilled in the art.

Disclosed herein are fluid delivery devices and processes for operating a fluid delivery devices. In some embodiments, a fluid delivery process may include an optical monitoring process configured to determine fluid delivery information to monitor fluid delivery, including tracking an amount of fluid delivered and/or an amount of fluid in a reservoir. In some embodiments, a fluid delivery device may include a fluid delivery mechanism configured to cause delivery of a fluid from a reservoir and into a wearer of the fluid delivery device. The fluid delivery device may include an optical monitoring system configured to monitor operation of the fluid delivery mechanism.

In some embodiments, the optical monitoring system may be configured to generate optical information that may be used to determine fluid delivery information. For example, the optical monitoring system may be configured to detect light (for instance, optical information) associated with (for instance, reflecting off of a surface of) a component (for instance, a plunger, a drive rod, a leadscrew, or associated component) of the fluid delivery mechanism that may be translated into a measurement of an amount of fluid in a reservoir (for instance, fluid delivery information).

In some embodiments, the fluid delivery device may include or may be a medicament delivery device. In various embodiments, the medicament delivery device may be configured to deliver insulin. For example, the medicament delivery device may be an automated insulin delivery (AID) device. Although insulin delivery devices are used in examples in the present disclosure, embodiments are not so limited as these are provided for illustrative purposes. Embodiments may operate with various types of fluid or drug delivery devices, drugs or other fluids, analytes, automated drug delivery applications, and/or analyte sensors in accordance with the teachings of the present disclosure.

Wearable insulin infusion devices, such as an AID device, are designed to dispense insulin into the subcutaneous space for patients requiring insulin therapy to maintain their blood sugar levels. Insulin infusion devices may include various types of pumps or pump systems. For example, an insulin infusion device may use a syringe-style pump (for instance, pumps with a plunger arranged within a reservoir configured to dispense insulin) must keep track of the motion of the plunger and ensure that it moves appropriately during pumping. Additionally, not all pumps can accurately measure how much insulin was initially loaded into the device, which may lead to uncertainty regarding the amount of fluid in the reservoir (and how much fluid has been dispensed, exactly when the reservoir will be completely empty, and/or the like).

Accordingly, some embodiments provide an optical monitoring system configured to monitor fluid delivery information, including, without limitation, an amount of fluid delivered and/or an amount of fluid in a reservoir. In some embodiments, an optical monitoring system may be configured to determine optical information based on the movement of an element of a fluid delivery mechanism. In some embodiments, the element may be a plunger, a rod (for instance, a driving rod), a wheel, a gear, and/or the like. In various embodiments, a portion of the element may be configured to differentially reflect light. In this manner, light may be directed onto the portion and detected by a sensor. Optical information in the form of different characteristics of the light reflecting off of different portions of the element may be used to determine a location, orientation, or other property of the element (for instance, a distance traveled by a plunger). This optical information may then be translated into fluid delivery information (for instance, a volume of fluid that has been dispensed from the reservoir, a fill level of the reservoir, and/or the like).

For example, in some embodiments, an optical monitoring system may use reflection of light interacting with a drive rod, drive tube or a plunger element to determine proper motion and/or for dead reckoning on remaining volume of drug in a reservoir. In various embodiments, the optical monitoring system may be based on a rotating element which conveys rotation to a leadscrew/nut mechanism (a leadscrew combined with a split nut) to advance a plunger. In some embodiments, the optical monitoring system may provide multiple confirmation signals per rotation and/or a single confirmation of the plunger drive element reaching a known position, for example, in a defined travel region, which may be used as a dead reckoning of the remaining units of insulin in the reservoir.

In various embodiments, the optical monitoring system may use light emitter/receiver elements. In one non-limiting example, a light emitter/receiver may use infrared (IR) light. In some embodiments, the optical monitoring system may use a stacked IR transmitter/receiver pair. In various embodiments, the optical monitoring system may use a narrow vertical fan beam such that the sensitivity of the detector is not substantially affected by slightly off-axis reflections to the receiver window. Such an ability to detect off-axis signals may be useful when dealing with the rounded surface that will be the reflecting surface for a sensor arrangement according to some embodiments. In other embodiments, a side-by-side configuration may be used for the sensor/emitter pair(s).

In various embodiments, a light transmitter and receiver may be positioned next to a drive tube of a fluid delivery mechanism, which engages with a leadscrew connected to a syringe plunger. As the drive tube rotates, so does the leadscrew, which causes the leadscrew to move linearly along a rotational axis, relative to the drive tube. As the leadscrew advances, so does a syringe plunger. In some embodiments, at the end of the lead screw, a splined feature may be configured to engage with the rotational motion of the drive tube, while also allowing linear motion of the leadscrew relative to the drive tube. The splined end of the leadscrew may also provide a reflective element that may be detected by a light sensor, for example, when the plunger reaches the dead reckoning point in the reservoir. The dead reckoning point refers to a point or position of the plunger, wherein the leadscrew is in a position, wherein the optical monitoring system can detect the position of the leadscrew. The position may be a position of the leadscrew along its longitudinal axis of movement or specific degree of axial rotation.

In some embodiments, a light transmitter and receiver may be positioned next to drive tube of a fluid delivery mechanism, which engages with a leadscrew connected to a syringe plunger, wherein the drive tube is rotatable about the leadscrew's axis and unmovable/fixed in axial direction. The leadscrew may comprise an external thread engaging with an internal thread of the drive tube. The drive tube may be configured to rotate to move the leadscrew in axial direction. When moved in axial direction due to the rotation of the drive tube, the leadscrew may be configured not to rotate. The leadscrew may be integrally formed with the syringe plunger, e.g. an insert-molded assembly. Accordingly, the element configured to reflect light may be or may be comprised in the drive tube.

In some embodiments, the reservoir and syringe plunger may have a non-circular, in particular elliptical, cross-section perpendicular to the axis of the leadscrew. The non-circular cross-section may prevent rotation of the plunger within the reservoir, which may also prevent rotation of the leadscrew.

The drive tube may be driven by a drive wheel, in particular wherein the drive wheel is attached to a clutch mechanism. The clutch mechanism may be disengaged from the drive wheel when filling the reservoir, to allow the leadscrew to move in axial direction away from the reservoir and may be engaged to the drive wheel to rotate the drive tube to advance the leadscrew in axial direction towards the reservoir.

In exemplary embodiments, several reflective inserts may be arranged adjacent to the light emitter and detector. In some embodiments, the reflective inserts may be or may include holes cut through them which allow the light emitter/detector pair to detect the presence of the opening. When the opening is directly in front of the emitter/detector pair (+/- a small angular window), the light beam path may pass through the window to the interior of the drive tube. Accordingly, some embodiments may provide a signal on the leading and trailing edge of each window in the drive tube. By measuring the angle, or angular increment count for the drive tube, the optical monitoring system may operate to determine if the drive tube is rotating the proper amount.

An optical monitoring system according to various embodiments may provide multiple technological advantages over conventional systems. In one non-limiting technological advantage, embodiments may provide for the effective, repeatable, and accurate control of fluid flow (and, therefore, medicament dosages) for wearable medicament delivery devices. In another non-limiting technological advantage, embodiments may provide a fluid flow control assembly or valve that is not susceptible (or less susceptible) to environmental conditions, such as temperature. In an additional non-limiting technological advantage, embodiments may provide an optical monitoring system capable of continuous measurement of fluid status (for instance, amount of fluid dispensed, reservoir volume, individual dosages, and/or the like). In a further non-limiting technological advantage, embodiments may provide a fluid flow control assembly or valve that limits or eliminates the need for additional devices (such as switch valves) that require power and other resources that degrade device efficiency and lifespan.

Other embodiments and technological advantages are contemplated in the present disclosure.

In this description, numerous specific details, such as component and system configurations, may be set forth in order to provide a more thorough understanding of the described embodiments. It will be appreciated, however, by one skilled in the art, that the described embodiments may be practiced without such specific details. Additionally, some well-known structures, elements, and other features have not been shown in detail, to avoid unnecessarily obscuring the described embodiments.

In the following description, references to "one embodiment," "an embodiment," "example embodiment," "various embodiments," etc., indicate that the embodiment(s) of the technology so described may include particular features, structures, or characteristics, but more than one embodiment may and not every embodiment necessarily does include the particular features, structures, or characteristics. Further, some embodiments may have some, all, or none of the features described for other embodiments.

As used in this description and the claims and unless otherwise specified, the use of the ordinal adjectives "first," "second," "third," etc. to describe an element merely indicate that a particular instance of an element or different instances of like elements are being referred to, and is not intended to imply that the elements so described must be in a particular sequence, either temporally, spatially, in ranking, or in any other manner.

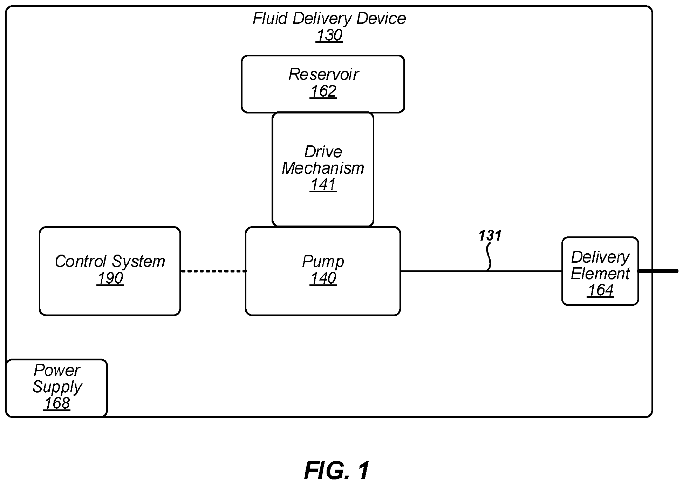

**FIG. 1** illustrates an example of an operating environment 100 that may be representative of some embodiments. As shown in FIG. 1, operating environment 100 may include a fluid delivery device 130. In various embodiments, fluid delivery device 130 may include a control system 190 that, in some embodiments, may be communicatively coupled to a computing device (not shown). Computing device may be or may include one or more logic devices, including, without limitation, a server computer, a client computing device, a personal computer (PC), a workstation, a laptop, a notebook computer, a smart phone, a tablet computing device, a personal diabetes management (PDM) device, a microcontroller (MCU) and/or the like.

In some embodiments, computing device may be in wired or wireless communication with fluid delivery device 130, for instance, via control system 190. For example, control system 190 may communicate via various wireless protocols, including, without limitation, Wi-Fi (i.e., IEEE 802.11), radio frequency (RF), Bluetooth^{™}, Zigbee^{™}, near field communication (NFC), Medical Implantable Communications Service (MICS), and/or the like. In another example, control system 190 may communicate via various wired protocols, including, without limitation, universal serial bus (USB), Lightning, serial, and/or the like. Although control system 190 is depicted as being within fluid delivery device 130, embodiments are not so limited. For example, in some embodiments, control system 190 and fluid delivery device 130 may be separate devices. In another example, some or all of the components of control system 190 may be included in fluid delivery device 130. For example, control system 190 may include processor circuitry, a memory unit, and/or the like. In some embodiments, each of control device 190 and fluid delivery device 130 may include a separate processor circuitry, memory unit, and/or the like capable of facilitating occlusion management processes according to some embodiments, either individually or in operative combination. Embodiments are not limited in this context.

Fluid delivery device 130 may be or may include a wearable automatic fluid delivery device directly coupled to a patient, for example, directly attached to the skin of the patient via an adhesive and/or other attachment component. In some embodiments, fluid delivery device 130 may be or may include a medicament delivery device configured to deliver a liquid medicament, drug, therapeutic agent, or other medical fluid to a patient. In some embodiments, fluid delivery device 130 may be included in a medicament delivery device configured to deliver a liquid medicament, drug, therapeutic agent, or other medical fluid to a patient. Non-limiting examples of medicaments may include insulin, glucagon or a glucagon-like peptide, pain relief drugs, hormones, blood pressure medicines, morphine, methadone, chemotherapy drugs, proteins, antibodies, and/or the like.

In some embodiments, fluid delivery device 130 may be or may include an automated insulin delivery (AID) device configured to deliver insulin (and/or other medication) to a patient. For example, fluid delivery device 160 may be or may include a device the same or similar to an OmniPod^{®} device or system provided by Insulet Corporation of Acton, Massachusetts, United States, for example, as described in United States Patent Nos. 7,303,549; 7,137,964; and/or 6,740,059, the contents of each of which is incorporated herein by reference in its entirety. Although an AID device and insulin are used in examples in the present disclosure, embodiments are not so limited, as fluid delivery device 130 may be or may include a device capable of storing and delivering any fluid including, without limitation, therapeutic agent, drug, medicine, hormone, protein, antibody, and/or the like.

Fluid delivery device 130 may include a delivery system having a number of components to facilitate automated delivery of a fluid to a patient, including, without limitation, a reservoir 162 for storing the fluid, a pump 140 for transferring the fluid from reservoir 162 and through a fluid path or conduit 131, and into the body of a patient via at least one delivery element 164, such as a needle and/or cannula, configured to be inserted through the skin of the patient. Embodiments are not limited in this context, for example, as delivery system 162 may include more or less components.

In some embodiments, fluid delivery device 130 may include a drive mechanism 141 configured to drive or otherwise case fluid to be released from reservoir 162, through fluid path 131, and out through delivery element 164. Drive mechanism 141 may operate using various elements, such as plungers, rods, screws, gears, nuts, activation elements (for instance, a motor, an SMA wire, servo elements, and/or the like), drive tubes, and/or the like.

In various embodiments, pump 140 may be a syringe-style pump. Accordingly, in some embodiments, drive mechanism 141 may be configured to operate with a syringe-style pump. Non-limiting examples of pumps and drive mechanisms may be the same or similar to configurations described in U.S. Patent Application No. 2005/0238507.

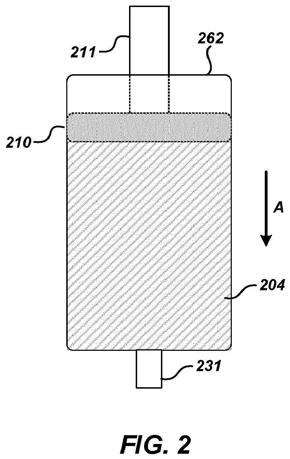

**FIG. 2** illustrates a drive mechanism for a fluid delivery device in accordance with the present disclosure. As shown in FIG. 2, a drug container or reservoir 262 can hold or store a liquid drug or other therapeutic agent 204. A plunger 210 can be used to expel liquid drug 204 from reservoir 262 for delivery to a patient. A force element 211 can provide a force on plunger 210 to drive or advance plunger 210 in a direction A to expel liquid drug 204 from reservoir 262 (e.g., to advance plunger 210 from a first position to a second position further into reservoir 262 to expel liquid drug 204). A drive mechanism may include additional components, such as a lead screw, drive tube, and/or the like.

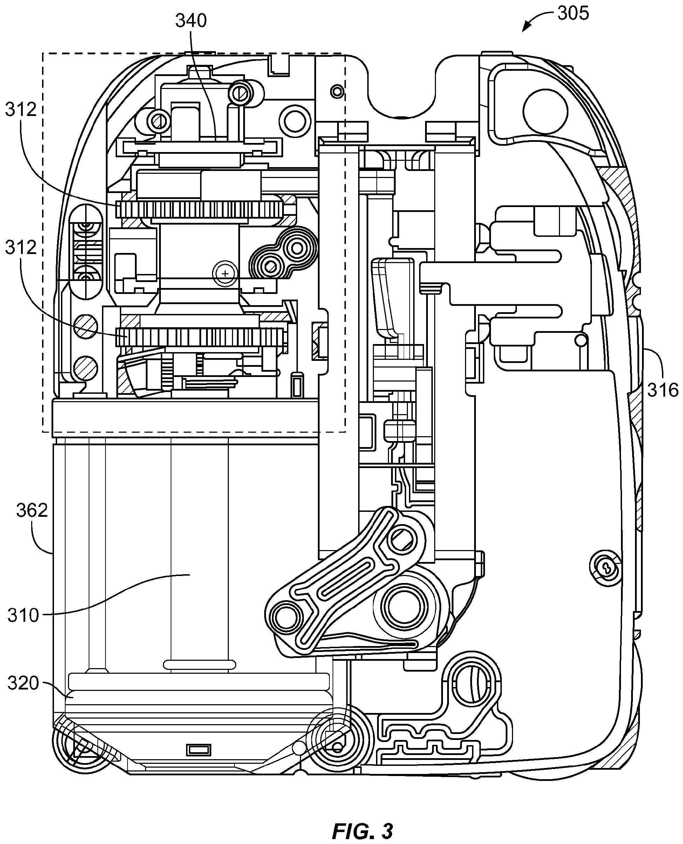

**FIG. 3** illustrates an exemplary wearable fluid delivery device in accordance with the present disclosure. In particular, FIG. 3 depicts a top-down view of a wearable fluid delivery device 305. As shown in FIG. 3 a wearable fluid delivery device 305 may include multiple systems to store and deliver a fluid to a patient. In some embodiments, wearable fluid delivery device 305 may include a pump 340. In exemplary embodiments, wearable fluid delivery device 305 may include a reservoir 362 for storing a fluid. Reservoir 362 may be in fluid communication with pump 340 for delivering the fluid to a patient via a needle. The pump 340 may be coupled to a drive element or a force element 310 that is further coupled to plunger 320. The drive or force element 310 may be operable to travel and apply a force to the plunger 320. The plunger 320 may be operable to travel within the reservoir 362 to expel fluid from the reservoir 362. In some embodiments, components of pump 305 may be arranged within one or more housings 316.

Although one type of pump is illustrated in FIG. 3, embodiments are not so limited, as any type of pump capable of operating according to some embodiments is contemplated in the present disclosure. Non-limiting examples of pumps may include positive displacement, syringe-style, reciprocating (diaphragm, piston, and/or the like), rotary motor, MEMS, piezoelectric, and/or the like. Embodiments are not limited in this context.

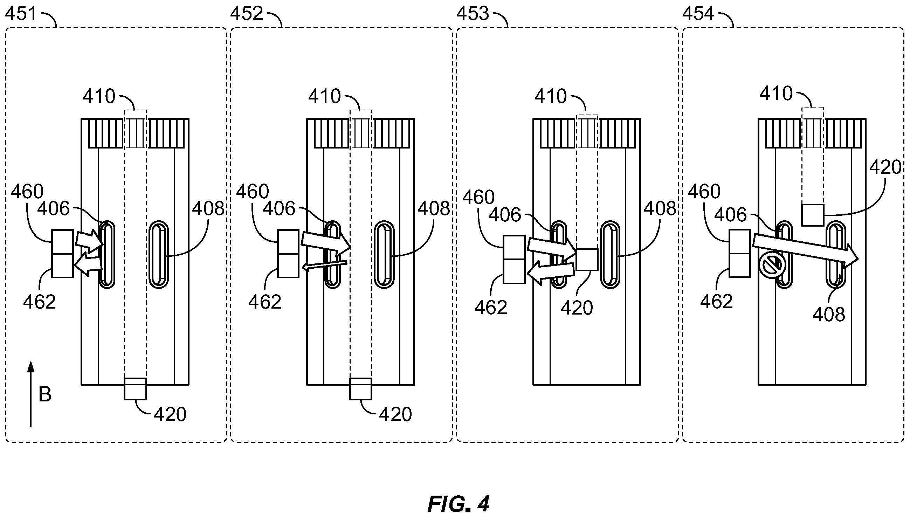

**FIG. 4** illustrates an example optical monitoring system in accordance with the present disclosure. As shown in FIG. 4, an optical monitoring system may include a light source 460 and a sensor 462 configured to receive light from light source 460. Sensor 462 may be configured to measure various properties of the light, such as intensity, brightness, wavelength, and/or the like. In some embodiments, the light may be IR light. In various embodiments, sensor 462 may be a photodiode. Although one light source 460 and sensor 462 are depicted in FIG. 4, embodiments are not so limited, as the optical monitoring system may include a plurality of light sources 460 and/or sensors 462. In some embodiments with a plurality of light sources 460 and/or sensors 462, multiple sensors 462 may be configured to receive light from one or more light sources 460, and the different light sources 460 may emit different types of light, which differences may be sensed by sensor 462.

As shown in FIG. 4, a drive mechanism may include a tube 406 having a drive element 410 (for instance, a plunger, lead screw, and/or the like) arranged therein. Drive element 410 is configured to travel in direction B (a fluid dispensing direction) to dispense fluid from a reservoir (not shown). The term "fluid dispensing direction" relates to a travel direction of the drive element 410, which when the drive element 410 travels in that direction results in fluid being dispensed from the fluid delivery device. In various embodiments, drive element 410 and/or tube 406 may have or may be coupled to various reflector elements 420. In some embodiments, reflector elements 420 may be used to indicate a position of drive element 410 and/or tube 406, including, for example, as depicted in FIG. 4, the end of drive element 410.

In some embodiments, there may be a plurality of reflector elements 420 along a length of the drive element 410, with a plurality of reflector elements 420 having different reflective properties so that they reflect light with different characteristics. For example, a first reflector element 420 may be arranged at a first section of drive element 410 or tube 406 and a second reflector element 420 may be arranged at a second section of drive element 410 or tube 406. The first reflector element 420 and the second reflector element 420 may have different reflective properties which may be detected, measured, and/or the like by sensor 462. In this manner, the optical monitoring system may determine when drive element 410 or tube 406 has reached the first second, the second section, and so on.

In various embodiments, tube 406 may include one or more slots, windows, or other openings 408 configured to allow light from source 460 to pass through tube 406 and become incident on drive element 410 within tube 406. The window may comprise a windowpane allowing light of at least one wavelength in the UV-(ultraviolet), visible- and/or IR- (infrared) spectrum to pass. The windowpane may comprise for example a glass, crystal or polymer transparent to one or more wavelengths in the aforementioned light spectra. In the following, the terms "opening 408" and "window 408" are used technically interchangeably. Any instance of the term "window 408" or "opening 408" shall be understood as "opening 408 or window 408". In addition, window 408 may allow light reflecting off of drive element 410 to pass through tube 406 and be received by sensor 462. In various embodiments, window 408 may include and operate as a reflective frame. For example, window 408 may be configured as a sensing window/frame, for instance, that is insert molded into drive tube 406. In some embodiments, a plurality of windows 408 may be arranged on tube 406. With a reflective frame, as each window 408 moves into rotational alignment with a light source 460, sensor 462 may detect that a "front" portion of window 408 has been rotated into position, an opening of window 408 has been rotated into position, and an "end" portion of window 408 has been rotated into position with respect to the light source 460 and sensor 462.

As shown in FIG. 4, light source 460 and sensor 462 may be arranged (or stacked) on or adjacent to each other. In various embodiments, light source 460 and sensor 462 may be arranged on the same side of tube 406.

Motion sensing by optical monitoring system may take places in various stages 451-454. For example, as drive tube 406 rotates, there may be little to no reflected signal until drive tube 406 rotates such that one of openings 408 or a frame of one of openings 408 reflects light from light source 460 onto sensor 462. If each opening 408 has a reflective frame, the signal to the detector may be elevated as long as sensor 462 is viewing the frame. As drive tube 406 advances, the signal will change when sensor 462 is viewing through opening 408 (and light source 460 is potentially reflecting off of something within window 408).

For example, at stage 451, light source 460 may not be lined up with window 408. Accordingly, light is reflecting off of an outside surface of tube 406. The characteristic of light reflecting off of the outside surface of tube (e.g., a light intensity) may be known or within a known range, threshold, and/or the like so that optical monitoring system may be able to determine that light source 460 is not aligned with window 408.

At stage 452, if drive element 410 is currently within (or in line with light source 460) opening or window 408, a different reflective signal may be provided (e.g., than at stage 451). The signal in stage 452 may be stronger or weaker than the signal in stage 451, depending on the amount of reflectivity of the drive element 410 and the tube 406. The system may be configured such that there is a detectable difference in the signal among stages 451-453, regardless of whether that difference is a weaker or stronger signal.

At stage 453, if the end of drive element 410 is in the window 408, a large signal may be detected because, for example, light is reflecting off of reflector element 420. At stage 454, if the end of drive element 410 has completely passed window 408, then there may be little to no signal (for instance, because light is not reflecting off of drive element 410 or reflector element 420). The optical information from last stage 454 may indicate that drive element 410 has passed the dead reckoning position and a known amount of insulin remains in the reservoir at that instant. Should this signal be received when the pump is initiating, it would be clear that the minimum amount of insulin needed to perform the dead reckoning was not used and there is no way to determine how much insulin is remaining in the pump. A pump control system program may then act accordingly to manage the condition of the fluid delivery device.

An advantage of using a reflecting configuration as depicted in FIG. 4 is that it provides measurement of the drive tube rotation at all times. As the windows pass by the detector, they can detect that the drive tube is in fact rotating. Otherwise, this must be inferred through a drive train which could itself not be functioning properly. Additionally, having the sensor and detector on the same side of the drive tube avoids the need for additional wiring or PCBA that is needed for a transmissive sensor.

More or fewer stages than those depicted in FIG. 4 may be used by optical monitoring system. In some embodiments, threshold values for light properties (intensity, wavelength, and/or the like) may be used by a pump control system or program to determine fluid delivery information. For example, light intensity at stage 453 may increase over a threshold indicating that the end of drive element 410 has been reached. In another example, light intensity at stage 454 may decrease below a threshold indicating that the end of drive element 410 has passed. Other combinations of thresholds, including in series (for instance, an increase over a threshold (e.g., stage 453) followed by a decrease below a threshold (e.g., stage 454), may be used in some embodiments to determine fluid delivery information.

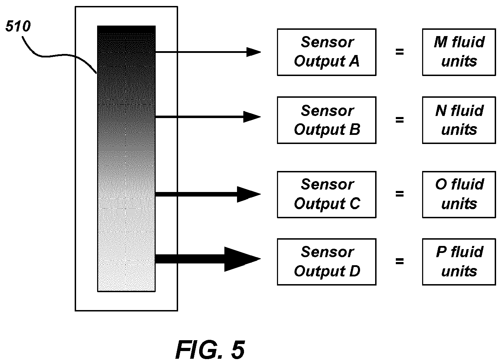

**FIG. 5** illustrates an example differential or light reflectance element in accordance with the present disclosure. As shown in FIG. 5, a drive mechanism element 510, such as a lead screw, plunger, and/or the like, may be formed of a material with different light reflective properties on the surface along its length thereof. For example, a reflective gradient may be arranged on a surface of element 510. In this manner, different portions of element 510 may have different light reflective properties so that sensor output may be used to determine which portion of element 510 is being measured. In various embodiments, the different sensor outputs may be used to determine an amount of dispensed fluid and/or an amount of fluid in a reservoir. For example, sensor output A associated with a first portion of element 510 may indicate M fluid units in reservoir; sensor output B associated with a first portion of element 510 may indicate N fluid units in reservoir; sensor output C associated with a first portion of element 510 may indicate O fluid units in reservoir; sensor output D associated with a first portion of element 510 may indicate P fluid units in reservoir; and so on. Many more than four differential values may be used, such as tens, hundreds, or even thousands of different reflective levels may be detected by sensor 460, Corresponding to a different position of drive element 410/510, which in turn corresponds to a different amount of fluid in the reservoir 362.

The gradient may be achieved via various techniques, such as a film or strip of material on element 510 (e.g., a lead screw) that gradually changed in color (or ink particles or etchant) along its length, such that a different reflectance is measured as the different colors (or particles or etchant) traverse the window.

In various embodiments, the use of a plurality of reflector elements (see, for example, FIG. 4) and/or a reflective gradient may be used to generate optical information that may be used by a control system or program to continuously determine fluid level status. In some embodiments, a "fuel gauge" or other GUI object may be used to visualize the fluid status, for example, on a computing device associated with a fluid delivery device. Thus, as fluid is inserted into reservoir 362, element 410/510 may traverse backwards, and sensor 462 may detect how far element 410/510 has moved, which corresponds to how far plunger 320 has moved in reservoir 362, which in turn corresponds to how much liquid drug has been inserted into reservoir 362. In this manner, the differential light reflectance element 510 may function as a "fuel gauge" to determine how much liquid drug has been inserted into reservoir 362, and thereafter how much liquid drug has been expelled from reservoir 362. The amount of reflectance expected along the linear length of element 510 may be stored in a memory (e.g., 623/653/673) and correspond to a position of element 410/510, which in turn correspond to an amount of liquid in reservoir 362. Thus, as a certain amount of reflectance is detected, this amount may be compared with values stored in memory and used to determine how much liquid drug is in reservoir 362. Such information may be communicated to the user (e.g., via a user interface) by communicating this data to controller 605, smart accessory device 607, or cloud-based services 611, which are described in further detail below. In this manner, the system 600 and the user may know how much drug has been inserted into reservoir 362 upon initial setup and how much drug remains in reservoir 362 as amounts of drug are gradually expelled from reservoir 362 during system use. Embodiments are not limited in this context.

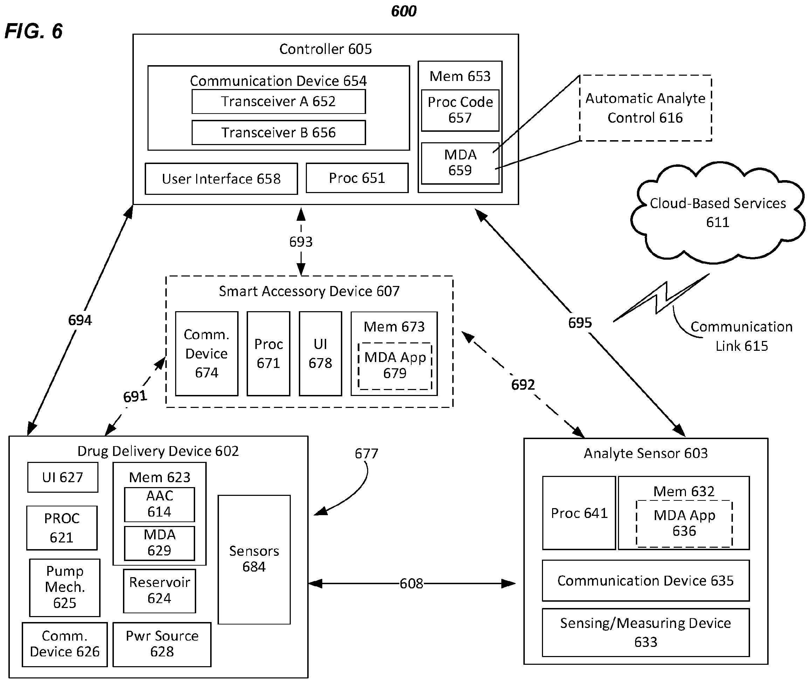

**FIG. 6** illustrates a functional block diagram of a system example suitable for implementing the example processes and techniques described herein.

The operating environment 600 may be or may include an automatic drug delivery system that may include components such as an automated drug delivery system that is configured to determine a drug dosage and deliver the dosage of the drug without any user interaction, or in some examples, limited user interaction, such as in response to a user pressing a button to indicate measurement of blood glucose or another analyte, or to input a number of carbohydrates the user is going to consume, or that the user is consuming a meal, or the like. "Drug delivery system environment" may refer to a computing and sensing environment that includes cloud based services, a drug delivery system (that may include a controller, a drug delivery device, and an analyte sensor) and optionally additional devices. The components of the drug delivery system environment may cooperate to provide present analyte measurement values or at least accurate estimates of present analyte measurement values to facilitate calculation of optimal drug dosages for a user.

The automated drug delivery system 600 may implement (and/or provide functionality for) a medication delivery algorithm, such as an artificial pancreas (AP) application, to govern or control automated delivery of a drug or medication, such as insulin, to a user (e.g., to maintain euglycemia - a normal level of glucose in the blood). The drug delivery system 600 may be an automated drug delivery system that may include a wearable automated drug delivery device 602, an analyte sensor 603, and a management device (for instance, a PDM, smart phone, table computing device, and/or the like) 605.

The system 600, in an optional example, may also include a smart accessory device 607, such as a smartwatch, a personal assistant device or the like, which may communicate with the other components of system 600 via either a wired or wireless communication links 691-593.

The management device 605 may be a computing device such as a smart phone, a tablet, a personal diabetes management device, a dedicated diabetes therapy management device, or the like. In an example, the management device (PDM) 605 may include a processor 651, a management device memory 653, a user interface 658, and a communication device 654. The management device 605 may contain analog and/or digital circuitry that may be implemented as a processor 651 for executing processes based on programming code stored in the management device memory 653, such as the medication delivery algorithm or application (MDA) 659, to manage a user's blood glucose levels and for controlling the delivery of the drug, medication, or therapeutic agent to the user as well as other functions, such as calculating carbohydrate-compensation dosage, a correction bolus dosage and the like as discussed above. The management device 605 may be used to program, adjust settings, and/or control operation of the wearable automatic drug delivery device 602 and/or the analyte sensor 603 as well as the optional smart accessory device 607.

The processor 651 may also be configured to execute programming code stored in the management device memory 653, such as the MDA 659. The MDA 659 may be a computer application that is operable to deliver a drug based on information received from the analyte sensor 603, the cloud-based services 611 and/or the management device 605 or optional smart accessory device 607. The memory 653 may also store programming code to, for example, operate the user interface 658 (e.g., a touchscreen device, a camera or the like), the communication device 654 and the like. The processor 651 when executing the MDA 659 may be configured to implement indications and notifications related to meal ingestion, blood glucose measurements, and the like. The user interface 658 may be under the control of the processor 651 and be configured to present a graphical user interface.

In a specific example, when the MDA 659 is an artificial pancreas (AP) application, the processor 651 is also configured to execute a diabetes treatment plan (which may be stored in a memory) that is managed by the MDA 659 stored in memory 653. In addition to the functions mentioned above, when the MDA 659 is an AP application, it may further provide functionality to enable the processor 651 to determine a carbohydrate-compensation dosage, a correction bolus dosage and determine a basal dosage according to a diabetes treatment plan. In addition, as an AP application, the MDA 659 provides functionality to enable the processor 651 to output signals to the wearable automatic drug delivery device 602 to deliver dosages according to some embodiments.

The communication device 654 may include one or more transceivers such as Transceiver A 652 and Transceiver B 656 and receivers or transmitters that operate according to one or more radio-frequency protocols. In the example, the transceivers 652 and 656 may be a cellular transceiver and a Bluetooth^{®} transceiver, respectively. For example, the communication device 654 may include a transceiver 652 or 656 configured to receive and transmit signals containing information usable by the MDA 659.

The wearable automatic drug delivery device 602, in the example system 600, may include a user interface 627, a controller 621, a drive mechanism 625, a communication device 626, a memory 623, a power source/energy harvesting circuit 628, device sensors 684, and a reservoir 624. The wearable automatic drug delivery device 602 may be configured to perform and execute the processes described in the examples of the present disclosure without input from the management device 605 or the optional smart accessory device 607. As explained in more detail, the controller 621 may be operable, for example, to determine an amount of insulin delivered, IOB, insulin remaining, and the like. The controller 621 alone may determine an amount of insulin delivered, IOB, insulin remaining, and the like, such as control insulin delivery, based on an input from the analyte sensor 604.

The memory 623 may store programming code executable by the controller 621. The programming code, for example, may enable the controller 621 to control expelling insulin from the reservoir 624 and control the administering of doses of medication based on signals from the MDA 629 or, external devices, if the MDA 629 is configured to implement the external control signals.

The reservoir 624 may be configured to store drugs, medications or therapeutic agents suitable for automated delivery, such as insulin, morphine, blood pressure medicines, chemotherapy drugs, or the like.

The device sensors 684 may include one or more of a pressure sensor, a power sensor, or the like that are communicatively coupled to the controller 621 and provide various signals. For example, the pressure sensor may be coupled to or integral with a needle/cannula insertion component (which may be part of the drive mechanism 625) or the like. In an example, the controller 621 or a processor, such as 651, may be operable to determine that a rate of drug infusion based on the indication of the fluid pressure. The rate of drug infusion may be compared to an infusion rate threshold, and the comparison result may be usable in determining an amount of insulin onboard (IOB) or a total daily insulin (TDI) amount.

In an example, the wearable automatic drug delivery device 602 includes a communication device 626, which may be a receiver, a transmitter, or a transceiver that operates according to one or more radio-frequency protocols, such as Bluetooth, Wi-Fi, a near-field communication standard, a cellular standard, or the like. The controller 621 may, for example, communicate with a personal diabetes management device 605 and an analyte sensor 603 via the communication device 626.

The wearable automatic drug delivery device 602 may be attached to the body of a user, such as a patient or diabetic, at an attachment location and may deliver any therapeutic agent, including any drug or medicine, such as insulin or the like, to a user at or around the attachment location. A surface of the wearable automatic drug delivery device 602 may include an adhesive to facilitate attachment to the skin of a user as described in earlier examples.

The wearable automatic drug delivery device 602 may, for example, include a reservoir 624 for storing the drug (such as insulin), a needle or cannula (not shown in this example) for delivering the drug into the body of the user (which may be done subcutaneously, intraperitoneally, or intravenously), and a drive mechanism 625 for transferring the drug from the reservoir 624 through a needle or cannula and into the user. The drive mechanism 625 may be fluidly coupled to reservoir 624, and communicatively coupled to the controller 621.

The wearable automatic drug delivery device 602 may further include a power source 628, such as a battery, a piezoelectric device, other forms of energy harvesting devices, or the like, for supplying electrical power to the drive mechanism 625 and/or other components (such as the controller 621, memory 623, and the communication device 626) of the wearable automatic drug delivery device 602.

In some examples, the wearable automatic drug delivery device 602 and/or the management device 605 may include a user interface 658, respectively, such as a keypad, a touchscreen display, levers, light-emitting diodes, buttons on a housing of the management device 605, a microphone, a camera, a speaker, a display, or the like, that is configured to allow a user to enter information and allow the management device 605 to output information for presentation to the user (e.g., alarm signals or the like). The user interface 658 may provide inputs, such as a voice input, a gesture (e.g., hand or facial) input to a camera, swipes to a touchscreen, or the like, to processor 651 which the programming code interprets.

When configured to communicate to an external device, such as the PDM 605 or the analyte sensor 604, the wearable automatic drug delivery device 602 may receive signals over the wired or wireless link 694 from the management device (PDM) 605 or 608 from the analyte sensor 604. The controller 621 of the wearable automatic drug delivery device 602 may receive and process the signals from the respective external devices as described with reference to the examples of the present disclosure as well as implementing delivery of a drug to the user according to a diabetes treatment plan or other drug delivery regimen.

The analyte sensor 603 may include a controller 631, a memory 632, a sensing/measuring device 633, a user interface 637, a power source/energy harvesting circuitry 634, and a communication device 635. The analyte sensor 603 may be communicatively coupled to the processor 651 of the management device 605 or controller 621 of the wearable automatic drug delivery device 602. The memory 632 may be configured to store information and programming code, such as an instance of the MDA 636.

The analyte sensor 603 may be configured to detect multiple different analytes, such as lactate, ketones, uric acid, sodium, potassium, alcohol levels or the like, and output results of the detections, such as measurement values or the like. The analyte sensor 603 may, in an example, be configured to measure a blood glucose value at a predetermined time interval, such as every 6 minutes, every cycle, or the like. The communication device 635 of analyte sensor 603 may have circuitry that operates as a transceiver for communicating the measured blood glucose values to the management device 605 over a wireless link 695 or with wearable automatic drug delivery device 602 over the wireless communication link 608. While called an analyte sensor 603, the sensing/measuring device 633 of the analyte sensor 603 may include one or more additional sensing elements, such as a glucose measurement element a heart rate monitor, a pressure sensor, or the like. The controller 631 may include discrete, specialized logic and/or components, an application-specific integrated circuit, a microcontroller or processor that executes software instructions, firmware, programming instructions stored in memory (such as memory 632), or any combination thereof.

Similar to the controller 621, the controller 631 of the analyte sensor 603 may be operable to perform many functions. For example, the controller 631 may be configured by the programming code stored in the memory 632 to manage the collection and analysis of data detected the sensing and measuring device 633.

Although the analyte sensor 603 is depicted in FIG. 6 as separate from the wearable automatic drug delivery device 602, in various examples, the analyte sensor 603 and wearable automatic drug delivery device 602 may be incorporated into the same unit. That is, in various examples, the sensor 603 may be a part of the wearable automatic drug delivery device 602 and contained within the same housing of the wearable automatic drug delivery device 602 (e.g., the sensor 603 or, only the sensing/measuring device 633 and memory storing related programming code may be positioned within or integrated into, or into one or more components, such as the memory 623, of the wearable automatic drug delivery device 602). In such an example configuration, the controller 621 may be able to implement the process examples of present disclosure alone without any external inputs from the management device 605, the cloud-based services 611, another sensor (not shown), the optional smart accessory device 607, or the like.

The communication link 615 that couples the cloud-based services 611 to the respective devices 602, 603, 605 or 607 of system 600 may be a cellular link, a Wi-Fi link, a Bluetooth link, or a combination thereof. Services provided by cloud-based services 611 may include data storage that stores anonymized data, such as blood glucose measurement values, historical IOB or TDI, prior carbohydrate-compensation dosage, and other forms of data. In addition, the cloud-based services 611 may process the anonymized data from multiple users to provide generalized information related to TDI, insulin sensitivity, IOB and the like.

The wireless communication links 608, 691, 692, 693, 694 and 695 may be any type of wireless link operating using known wireless communication standards or proprietary standards. As an example, the wireless communication links 608, 691, 692, 693, 694 and 695 may provide communication links based on Bluetooth^{®}, Zigbee^{®}, Wi-Fi, a near-field communication standard, a cellular standard, or any other wireless protocol via the respective communication devices 654, 674, 626 and 635.

Software related implementations of the techniques described herein may include, but are not limited to, firmware, application specific software, or any other type of computer readable instructions that may be executed by one or more processors. The computer readable instructions may be provided via non-transitory computer-readable media. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

In addition, or alternatively, while the examples may have been described with reference to a closed loop algorithmic implementation, variations of the disclosed examples may be implemented to enable open loop use. The open loop implementations allow for use of different modalities of delivery of insulin such as smart pen, syringe or the like. For example, the disclosed AP application and algorithms may be operable to perform various functions related to open loop operations, such as the generation of prompts requesting the input of information such as weight or age. Similarly, a dosage amount of insulin may be received by the AP application or algorithm from a user via a user interface. Other open-loop actions may also be implemented by adjusting user settings or the like in an AP application or algorithm.

Some examples of the disclosed device or processes may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

Program aspects of the technology may be thought of as "products" or "articles of manufacture" typically in the form of executable code and/or associated data that is carried on or embodied in a type of non-transitory, machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The present disclosure furthermore relates to computer programs comprising instructions (also referred to as computer programming instructions) to perform the aforementioned functionalities. The instructions may be executed by a processor. The instructions may also be performed by a plurality of processors for example in a distributed computer system. The computer programs of the present disclosure may be for example preinstalled on, or downloaded to the medicament delivery device, management device, fluid delivery device, e.g. their storage.

The foregoing description of examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

Although the present invention is defined in the attached claims, it should be understood that the present invention can also (alternatively) be defined in accordance with the following embodiments:
1. A fluid delivery device, comprising:
   a reservoir storing a fluid;
   a pump fluidically coupled to the reservoir, the pump comprising a drive mechanism to force the fluid from the reservoir; and
   an optical monitoring system comprising:
      at least one light source operative to emit light incident on at least one element of the drive mechanism; and
      at least one sensor configured to receive the light reflecting off of the at least one element, wherein the at least one light source and the at least one sensor are arranged on a same side of the at least one element.
2. The fluid delivery device of embodiment 1, the pump comprising a syringe-style pump.
3. The fluid delivery device of embodiment 1, the at least one element comprising a lead screw.
4. The fluid delivery device of embodiment 3, the lead screw arranged within a drive tube configured to rotate the lead screw, causing the lead screw to move in a fluid dispensing direction,
   wherein the drive tube comprises at least one window arranged to allow the light from the light source to travel into the drive tube and be incident on the lead screw.
5. The fluid delivery device of embodiment 1, the at least one light source comprising an infrared (IR) light source.
6. The fluid delivery device of embodiment 1, the at least one light source and the at least one sensor arranged in a stacked configuration.
7. The fluid delivery device of embodiment 1, comprising at least one reflector element arranged on the at least one element, the at least one reflector element configured to reflect light with a different characteristic than the at least one element.
8. The fluid delivery device of embodiment 7, the at least one characteristic comprising at least one of intensity, frequency, or wavelength.
9. The fluid delivery device of embodiment 7, the at least one reflector element arranged on a portion of the at least one element to indicate that the at least one element has traveled a known distance.
10. The fluid delivery device of embodiment 1, the at least one element having at least a portion with a reflective gradient configured to reflect light with a different characteristic along a length of the portion of the at least one element.
11. The fluid delivery device of embodiment 10, the reflective gradient configured to indicate a position of the at least one element to provide a fill level of the reservoir.
12. The fluid delivery device of embodiment 1, the fluid delivery device comprising a wearable insulin pump.
13. An apparatus, comprising:
   at least one processor;
   a memory coupled to the at least one processor, the memory comprising instructions that, when executed by the at least one processor, cause the at least one processor to:
      receive optical information from an optical monitoring system for a fluid delivery device, the optical monitoring system comprising:
      at least one light source operative to emit light incident on at least one element of a drive mechanism configured to dispense fluid from a reservoir of the fluid delivery device, and
      at least one sensor configured to receive the light reflecting off of the at least one element, wherein the at least one light source and the at least one sensor are arranged on a same side of the at least one element and the at least one element has at least one of a reflector element or a reflective gradient arranged on a surface of the at least one element configured to reflect light with a different characteristic,
   determine a position of the at least one element based on the optical information, and
   determine a fluid level of the reservoir based on the position.

## Claims

1. A fluid delivery device, comprising:
a reservoir storing a fluid;
a pump fluidically coupled to the reservoir, the pump comprising a drive mechanism to force the fluid from the reservoir; and
an optical monitoring system comprising:
at least one light source operative to emit light incident on at least one element of the drive mechanism; and
at least one sensor configured to receive the light reflecting off of the at least one element, wherein the at least one light source and the at least one sensor are arranged on a same side of the at least one element.

2. The fluid delivery device of claim 1, the pump comprising a syringe-style pump.

3. The fluid delivery device of claim 1 or 2, the at least one element comprising a lead screw.

4. The fluid delivery device of claim 3, the lead screw arranged within a drive tube configured to rotate the lead screw, causing the lead screw to move in a fluid dispensing direction,
wherein the drive tube comprises at least one window arranged to allow the light from the light source to travel into the drive tube and be incident on the lead screw, in particular wherein the lead screw is or comprises the at least one element.

5. The fluid delivery device of claim 1 or 2, the lead screw arranged within a drive tube, wherein the drive tube is configured to rotate to move the leadscrew in axial direction,
wherein the drive tube comprises the at least one element,
or wherein the drive tube comprises at least one window arranged to allow the light from the light source to travel into the drive tube and be incident on the lead screw, in particular wherein the lead screw is or comprises the at least one element.

6. The fluid delivery device of any one of claims 1 to 5, the at least one light source comprising an infrared (IR) light source.

7. The fluid delivery device of any one of claims 1 to 6, the at least one light source and the at least one sensor arranged in a stacked configuration.

8. The fluid delivery device of any one of claims 1 to 7, comprising at least one reflector element arranged on the at least one element, the at least one reflector element configured to reflect light with a different characteristic than the at least one element.

9. The fluid delivery device of claim 8, the at least one characteristic comprising at least one of intensity, frequency, or wavelength.

10. The fluid delivery device of claim 7 or 9, the at least one reflector element arranged on a portion of the at least one element to indicate that the at least one element has traveled a known distance.

11. The fluid delivery device of any one of claims 1 to 10, the at least one element having at least a portion with a reflective gradient configured to reflect light with a different characteristic along a length of the portion of the at least one element.

12. The fluid delivery device of claim 11, the reflective gradient configured to indicate a position of the at least one element to provide a fill level of the reservoir.

13. The fluid delivery device of any one of claims 1 to 12, the fluid delivery device comprising a wearable insulin pump.

14. A method comprising the following steps:
receive optical information from an optical monitoring system for a fluid delivery device, the optical monitoring system comprising:
at least one light source operative to emit light incident on at least one element of a drive mechanism configured to dispense fluid from a reservoir of the fluid delivery device, and
at least one sensor configured to receive the light reflecting off of the at least one element, wherein the at least one light source and the at least one sensor are arranged on a same side of the at least one element and the at least one element has at least one of a reflector element or a reflective gradient arranged on a surface of the at least one element configured to reflect light with a different characteristic;
determine a position of the at least one element based on the optical information, and
determine a fluid level of the reservoir based on the position.

15. A computer program comprising instructions for the following steps:
receive optical information from an optical monitoring system for a fluid delivery device, the optical monitoring system comprising:
at least one light source operative to emit light incident on at least one element of a drive mechanism configured to dispense fluid from a reservoir of the fluid delivery device, and
at least one sensor configured to receive the light reflecting off of the at least one element, wherein the at least one light source and the at least one sensor are arranged on a same side of the at least one element and the at least one element has at least one of a reflector element or a reflective gradient arranged on a surface of the at least one element configured to reflect light with a different characteristic;
determine a position of the at least one element based on the optical information, and
determine a fluid level of the reservoir based on the position.
